# EUROPÄISCHE PATENTSCHRIFT

(11) **EP 3 512 592 B1**
(45) Veröffentlichungstag und Bekanntmachung des Hinweises auf die Patenterteilung: **31.03.2021**
(21) Anmeldenummer: 17797228.8
(22) Anmeldetag: 14.09.2017
(51) Int. Cl.: A61M 25/10, A61M 25/00

(54) **KATHETER ZUR APPLIKATION EINES MEDIUMS IN DAS MITTELOHR**
CATHETER FOR APPLYING A MEDIUM INTO THE MIDDLE EAR
CATHÉTER SERVANT À ADMINISTRER UN FLUIDE DANS L'OREILLE MOYENNE

(30) Priorität: 16.09.2016 DE 102016117460
(43) Veröffentlichungstag der Anmeldung: 24.07.2019
(73) Patentinhaber: Spiggle, David, 53819 Neunkirchen-Seelscheid (DE); Theis, Detlef, 51545 Waldbröl (DE)
(72) Erfinder: Spiggle, David, 53819 Neunkirchen-Seelscheid (DE); Theis, Detlef, 51545 Waldbröl (DE)
(74) Vertreter: Seyer, Roman
(86) Internationale Anmeldenummer: PCT/DE2017/100777
(87) Internationale Veröffentlichungsnummer: WO 2018/050165

(56) Entgegenhaltungen:
- WO-A1-00/04854
- WO-A2-2009/157884
- CN-U- 203 029 796
- US-A1- 2006 095 066
- US-A1- 2006 106 361
- US-A1- 2013 274 715

## Beschreibung

Die Erfindung betrifft einen Katheter zur Applikation eines Mediums in das Mittelohr.

Das Mittelohr ist über die Eustachi-Röhre mit dem Nasen- und Rachenraum verbunden, wobei die Eustachi-Röhre den Druckausgleich zwischen diesen Bereichen ermöglicht. Da das Mittelohr durch die enge Eustachi-Röhre nur schwer zugänglich ist, werden Applikationen ins Mittelohr üblicherweise mittels Injektionen durch das Trommelfell vorgenommen.

Applikationskatheter sind im Stand der Technik bekannt. Diese im Stand der Technik bekannten Applikationskatheter werden vorwiegend in der Urologie, Radiologie und weiteren medizinischen Bereichen verwendet, eignen sich jedoch nicht zur Applikation eines Mediums in das Mittelohr.

Nachteilig bei den aus dem Stand der Technik bekannten Applikationskathetern ist, dass diese nicht genau platziert werden können, dass sie die Gehörknöchelchen im Mittelohr schädigen können, dass sie die Eustachi-Röhre nicht abdichten und dass sie, wenn überhaupt, nur die Applikationen eines geringen Volumens an Medium ermöglichen Siehe, z.B. WO2009157884 A2 und US2006106361 A1.

Aufgabe der Erfindung ist, einen Katheter gemäß Anspruch 1 sowie der entsprechenden Unteransprüche bereitzustellen, der eine exakte Platzierung des distalen Endes des Katheters innerhalb der Paukenhöhle ermöglicht, der das Risiko einer Gehörknöchelchenschädigung minimiert, der die Applikation eines Mediums ermöglicht und der ein Abfließen des Mediums über die Eustachi-Röhre verhindert.

Zur Lösung der Aufgabe ist ein Katheter zur Applikation eines Mediums in das Mittelohr vorgesehen, umfassend einen Schaft mit einem distalen und einem proximalen Ende, einen inflatierbaren ersten Ballon, wobei das Innere des ersten Ballons mit einem ersten Inflationslumen in Verbindung steht, einen inflatierbaren zweiten Ballon, der distal zum ersten Ballon angeordnet ist, wobei das Innere des zweiten Ballons mit einem zweiten Inflationslumen in Verbindung steht, ein Applikationslumen mit einer Eintrittsöffnung, einer Austrittsöffnung und einem Mittelabschnitt, der sich von der Eintrittsöffnung bis zur Austrittsöffnung erstreckt, wobei die Eintrittsöffnung außerhalb des Schafts angeordnet ist, sich der Mittelabschnitt zumindest teilweise durch den Schaft erstreckt und die Austrittsöffnung distal des proximalen Endes des zweiten Ballons angeordnet ist, und ein Entlüftungslumen mit einer Eintrittsöffnung, einer Austrittsöffnung und einem Mittelabschnitt, der sich von der Eintrittsöffnung bis zur Austrittsöffnung erstreckt, wobei die Eintrittsöffnung distal des proximalen Endes des zweiten Ballons angeordnet ist, sich der Mittelabschnitt zumindest teilweise durch den Schaft erstreckt und die Austrittsöffnung außerhalb des Schafts angeordnet ist.

Die mit der Erfindung erzielten Vorteile bestehen unter anderem darin, dass der erste, proximale Ballon nach Inflation die Eustachi-Röhre abdichtet, sodass das applizierte Medium im Mittelohr verbleibt. Zudem wird der Katheter durch den diesen Ballon in der seiner Lage stabilisiert. Der inflatierte zweite, distale Ballon verhindert, dass das distale Ende des Katheters durch die Inflation des proximalen Ballons aus dem Mittelohr zurück in die Eustachi-Röhre gezogen wird und schützt zudem die Gehörknöchelchen vor Schädigungen, beispielsweise durch das distale Ende des Schafts. Darüber hinaus dichtet der distale Ballon ebenfalls die Eustachi-Röhre ab. Mittels des Applikationslumens kann ein Medium appliziert werden. Überraschenderweise wurde ferner festgestellt, dass die Applikation von Medien erheblich erleichtert ist, wenn ein Entlüftungslumen vorhanden ist, das die Entlüftung des Mittelohres während der Applikation gewährleistet. Weitere Vorteile der Erfindung ergeben sich aus den Unteransprüchen.

Der Katheter ist zur Applikationen von Medien, insbesondere von Flüssigkeiten oder Suspensionen ins Mittelohr, insbesondere an die Membranen, die zum Innenohr führen, vorgesehen. Hierfür wird der Katheter, mit dem distalen Ende des Schaftes voran, mittels eines Einführinstrumentes aus dem pharyngealen Tubenostium durch die Eustachi-Röhre geschoben bis das distale Ende des Schaftes einschließlich des zweiten, distalen Ballons die Paukenhöhle erreicht hat. Der erste, proximale Ballon befindet sich in dieser Position zumindest teilweise innerhalb der Eustachi-Röhre. In dieser Position wird der Katheter durch Inflatieren des ersten Ballons und des zweiten Ballons fixiert und das Applikationsmedium durch das Applikationslumen injiziert, während Luft durch das Entlüftungslumen entweichen kann. Das Einführinstrument ist über das proximale Schaftende wieder entfernbar. Während der Applikation ist es vorteilhaft, wenn der Kopf des Patienten seitlich gelagert wird, sodass das zu behandelnde Ohr mit der Ohrmuschel in Richtung des Bodens zeigt. Die Applikation sowie Entlüftung erfolgt somit nach dem "Duschkopfprinzip".

Erfindungsgemäß ist somit unter dem distalen Ende des Schafts, das Ende zu verstehen, welches zuerst in die Eustachi-Röhre geschoben wird. Demzufolge wird der Ballon, der den geringsten Abstand zum distalen Ende des Schafts aufweist, als distaler Ballon bezeichnet. Das proximale Ende des Schafts bildet folglich das dem distalen Ende gegenüberliegende andere Ende des Schafts. Als proximal wird erfindungsgemäß ein Gegenstand bezeichnet, wenn er vom jeweiligen Bezugspunkt in Richtung des proximalen Schaftendes angeordnet ist. Als distal wird erfindungsgemäß ein Gegenstand bezeichnet, wenn er vom jeweiligen Bezugspunkt in Richtung des distalen Schaftendes angeordnet ist.

Der Katheter umfasst erfindungsgemäß somit einen Schaft mit einem distalen und einem proximalen Ende. Der Schaft umhüllt zumindest einen Abschnitt der Lumen des Katheters.

Der Katheter umfasst ferner einen inflatierbaren ersten Ballon. Das Innere des ersten Ballons steht mit einem ersten Inflationslumen in Verbindung. Zudem umfasst der Katheter einen inflatierbaren zweiten Ballon, dessen Inneres mit einem zweiten Inflationslumen in Verbindung steht. Der zweite Ballon ist distal zum ersten Ballon angeordnet.

Der erste und der zweite Ballon des erfindungsgemäßen Katheters können durch Einleiten von Fluiden, bevorzugt Flüssigkeiten, besonders bevorzugt Salzlösungen, insbesondere isotonischen NaCI-Lösung (0,9%), durch die mit den Ballons verbundenen Lumen, erfindungsgemäß als Inflationslumen bezeichnet, inflatiert, d.h. expandiert, werden. Zur Deflation können die Inflationsmedien durch diese mit den Ballons verbundenen Lumen wieder ausgelassen werden.

Das erste und das zweite Inflationslumen erstrecken sich vom ersten beziehungsweise zweiten Ballon bis zu ihren proximalen Öffnungen, die außerhalb des Schaftes angeordnet sind. Das erste und das zweite Inflationslumen können sich, innerhalb oder außerhalb des Schaftes, zu einem gemeinsamen Inflationslumen vereinen, sodass sie eine gemeinsame proximale Öffnung aufweisen. Bevorzugt verlaufen das erste und das zweite Inflationslumen oder das gemeinsame Inflationslumen von dem ersten und zweiten Ballons innerhalb des Schaftes in Richtung des proximalen Endes des Schaftes und sind durch mindestens eine Schaftöffnung, bevorzugt durch mindestens eine Schaftöffnung am proximalen Ende des Schaftes, aus dem Schaft hinaus geführt.

Das Applikationslumen weist eine Eintrittsöffnung, eine Austrittsöffnung und einen Mittelabschnitt, der sich von der Eintrittsöffnung bis zur Austrittsöffnung erstreckt, auf. Die Eintrittsöffnung ist außerhalb des Schafts angeordnet. Ausgehend von der Eintrittsöffnung verläuft der Mittelabschnitt durch eine Schaftöffnung, bevorzugt am proximalen Ende des Schaftes, in das Schaftinnere, der Mittelabschnitt erstreckt sich innerhalb des Schaftes in Richtung des distalen Endes des Schaftes und tritt durch eine Schaftöffnung aus dem Schaftinneren wieder aus und endet in der Austrittsöffnung, die distal des proximalen Endes des zweiten Ballons angeordnet ist. Die zu applizierenden Medien können in die Eintrittsöffnung gegeben werden, verlaufen durch den Mittelabschnitt und treten an der Austrittsöffnung wieder aus dem Lumen aus.

Als Medien kommen hierfür erfindungsgemäß bevorzugt Flüssigkeiten und Suspensionen in Betracht. Besonders bevorzugt sind biologisch aktive Verbindungen, ganz besonders bevorzugt Medikamente.

Das Entlüftungslumen weist eine Eintrittsöffnung, eine Austrittsöffnung und einen Mittelabschnitt, der sich von der Eintrittsöffnung bis zur Austrittsöffnung erstreckt, auf. Die Austrittsöffnung ist außerhalb des Schafts angeordnet. Ausgehend von der Austrittsöffnung verläuft der Mittelabschnitt durch eine Schaftöffnung, bevorzugt am proximalen Ende des Schaftes, in das Schaftinnere, der Mittelabschnitt erstreckt sich weiter innerhalb des Schaftes in Richtung des distalen Endes des Schaftes und tritt durch eine Schaftöffnung aus dem Schaftinneren wieder aus und endet in der Eintrittsöffnung, die distal des proximalen Endes des zweiten Ballons angeordnet ist.

Das Entlüftungslumen dient zur Entlüftung des Mittelohres während der Applikation und sorgt dafür, dass im Innenohr kein Überdruck entsteht, der die Applikation des Applikationsmediums verhindert oder Bestandteile des Mittelohres schädigt. Durch dieses Entlüftungslumen kann somit Luft, Applikationsmedium oder anderweitige im Mittelohr befindliche Flüssigkeiten in die Eintrittsöffnung eintreten, durch den Mittelabschnitt zur Austrittsöffnung gelangen und durch die Austrittsöffnung entweichen.

Die Austrittsöffnung des Applikationslumens und die Eintrittsöffnung des Entlüftungslumens sind in einem Bereich des Schaftes angeordnet, der sich vom proximalen Ende des zweiten, d.h. distalen, Ballons bis zum distalen Ende des Schaftes erstreckt. Somit enden das Applikationslumen und das Entlüftungslumen auf Höhe des zweiten Ballons oder distal davon.

Das erste Inflationslumen, das zweite Inflationslumen oder das gemeinsame Inflationslumen können in dem Bereich, der sich außerhalb des Schaftes befindet, ebenso wie das Applikationslumen und das Entlüftungslumen in den Bereichen, die sich außerhalb des Schaftes in Richtung der Eintrittsöffnung im Falle des Applikationslumens und in Richtung der Austrittsöffnung im Falle des Entlüftungslumen erstrecken, eine Vorrichtung, beispielsweise ein Ventil, einen Schieber, eine Klappe oder einen Hahn, aufweisen, mit der die Durchflussmenge regelbar ist. Alle Lumen können in diesen Bereichen außerhalb des Schaftes ebenso eine Anschlussmöglichkeit für Katheterkupplungen aufweisen.

Neben den zuvor beschriebenen ersten Inflationslumen, zweiten Inflationslumen, Applikationslumen und Entlüftungslumen kann der Katheter zudem weitere Lumen umfassen. Ebenso ist es möglich, dass der erfindungsgemäße Katheter weitere Ballons neben dem ersten und zweiten Ballon umfasst.

In einer vorteilhaften Ausgestaltung der Erfindung stehen das erste Inflationslumen und das zweite Inflationslumen nicht miteinander in Fluidverbindung. Demnach sind der erste und der zweite Ballon mittels je eines separaten Lumens inflatierbar. Dies ermöglicht, dass zunächst der zweite Ballon und anschließend der erste Ballon inflatiert werden können, wodurch das distale Ende des Schaftes nicht in die Eustachi-Röhre zurückrutschen kann, sobald der proximale, erste Ballon inflatiert ist.

In einer vorteilhaften Ausgestaltung der Erfindung weist der Katheter somit insgesamt vier Lumen auf. Mittels des ersten und des zweiten Inflationslumens können der erste beziehungsweise der zweite Ballon separat inflatiert werden, mittels des Applikationslumens können Medien appliziert werden und mittels des Entlüftungslumens kann das Mittelohr während der Applikation entlüftet werden.

Erfindungsgemäß sind die Austrittsöffnung des Applikationslumens und die Eintrittsöffnung des Entlüftungslumens am distalen Ende des Schafts angeordnet. In einer solchen erfindungsgemäßen Ausgestaltung endet der Mittelabschnitt dieser Lumen in Schaftöffnungen am distalen Schaftende. Durch diese Anordnung wird gewährleitstet, dass, für den Fall, dass das zu behandelnde Ohr des Patienten während oder nach der Applikation des Mediums nicht mit der Ohrmuschel in Richtung des Bodens zeigt und sich das Medium somit in der Paukenhöhle um den zweiten, distalen Ballon sammelt, dieses gesammelte Medium nicht direkt durch das Entlüftungslumen beziehungsweise das Applikationslumen entweichen kann.

In einer vorteilhaften Ausgestaltung der Erfindung verlaufen das Applikationslumen und das Entlüftungslumen von den Schaftöffnungen am distalen Schaftende zum proximalen Schaftende innerhalb des Schafts, und enden mit der Eintrittsöffnung, im Falle des Applikationslumens, bzw. mit der Austrittsöffnung, im Falle des Entlüftungslumens, außerhalb des proximalen Schaftendes, wobei die Austrittsöffnung, im Falle des Entlüftungslumens, beziehungsweise die Eintrittsöffnung, im Falle des Applikationslumens, an den Schaftöffnungen am distalen Schaftende angeordnet sind.

In einer vorteilhaften Ausgestaltung der Erfindung ist der distale, erfindungsgemäß als zweiter Ballon bezeichnete, Ballon am distalen Ende des Schafts angeordnet. In einer solchen vorteilhaften Ausgestaltung befinden sich somit die Austrittsöffnung des Applikationslumens und die Eintrittsöffnung des Entlüftungslumens als auch der zweite Ballon am distalen Ende des Schafts. Ein Ballon gilt erfindungsgemäß als am distalen Ende des Schafts angeordnet, wenn er sich im inflatierten Zustand zumindest mit einem Ende entlang der Längsachse des Schafts bis zum distalen Ende des Schafts erstreckt. Eine solche Ausgestaltung hat den Vorteil, dass, aufgrund des distal angeordneten Ballons, keine Katheterbestandteile hervorstehen, die das Mittelohr schädigen könnten.

In einer vorteilhaften Ausgestaltung der Erfindung umhüllen der erste und der zweite Ballon die Mantelfläche des Schaftes in dem Bereich, in dem sie sich entlang der Längsachse des Schaftes erstrecken, vollständig.

Überraschenderweise wurde festgestellt, dass die Applikation eines Medium in das Mittelohr erheblich erleichtert ist, wenn das Entlüftungslumen einen größeren Durchmesser als das Applikationslumen aufweist. In einer vorteilhaften Ausgestaltung der Erfindung weist das Entlüftungslumen daher einen größeren Durchmesser als das Applikationslumen auf. Bevorzugt ist der Durchmesser des Entlüftungslumens um mindestens 5%, besonders bevorzugt um mindestens 10% und ganz besonders bevorzugt um mindestens 20 % größer als der Durchmesser des Applikationslumens. In einer vorteilhaften Ausgestaltung der Erfindung weisen die Katheterlumen je einen Durchmesser zwischen 0,05-0,5 mm auf.

In einer vorteilhaften Ausgestaltung der Erfindung beträgt der Schaftdurchmesser weniger als 2 mm, bevorzugt weniger als 1,5 mm, besonders bevorzugt weniger als 1,2 mm, insbesondere weniger als 1 mm. Erfindungsgemäß wird unter Schaftdurchmesser der Durchmesser senkrecht zur Längsachse des Schaftes verstanden. Ein Katheter mit solch einem geringen Schaftdurchmesser gewährleistet, dass der Katheter ohne großen Druck eingeführt und durch die Eustachi-Röhre geschoben werden kann.

In einer vorteilhaften Ausgestaltung der Erfindung handelt es sich bei dem ersten und/oder zweiten Ballon um einen Niederdruckballon. Erfindungsgemäß umfasst der Katheter somit einen ersten und/oder zweiten Ballon, der mit weniger als 1 bar, bevorzugt weniger als 0,75 bar, ganz besonders bevorzugt weniger als 0,5 bar Überdruck inflatierbar ist. Derartige Niederdruckballons haben gegenüber Hochdruckballons den Vorteil, dass sie die Schleimhaut der Eustachi-Röhre besser vor Schädigungen schützen, die Eustachi-Röhre aufgrund der erhöhten Flexibilität der Ballons besser abdichten und eine ausreichende Stabilität gewährleisten.

In einer vorteilhaften Ausgestaltung der Erfindung weist der erste Ballon im inflatierten Zustand eine Länge von 5 bis 30 mm, bevorzugt 8 bis 20 mm, besonders bevorzugt 10 bis 16 mm, und/oder einen Durchmesser von 3 bis 12 mm, bevorzugt von 5-10 mm, besonders bevorzugt 7-8 mm auf. In einer vorteilhaften Ausgestaltung der Erfindung weist der zweite Ballon im inflatierten Zustand eine Länge von 2 bis 8 mm, bevorzugt von 4 bis 6 mm, und/oder einen Durchmesser von 2 bis 8 mm, bevorzugt 4 bis 6 mm auf. Die Länge der Ballons wird erfindungsgemäß parallel zur Längsachse des Katheters gemessen, während der Durchmesser senkrecht zur Längsachse des Katheters gemessen wird.

In einer vorteilhaften Ausgestaltung der Erfindung ist der erste Ballon 3-10 mm, bevorzugt 5-8 mm, von dem zweiten Ballon beabstandet. Zur Bestimmung des Abstandes wird erfindungsgemäß der Abstand zwischen dem distalen Ende des ersten Ballons und dem proximalen Ende des zweiten Ballons im inflatierten Zustand gemessen. In einer solchen Ausgestaltung der Erfindung sind die Ballons somit durch den Schaft, umfassend das Applikationslumen, das Entlüftungslumen, und das Inflationslumen für den zweiten Ballon voneinander getrennt. Auf diese Weise kann der Katheter sowohl gut in der Applikationsposition fixiert werden als auch die Eustachi-Röhre abdichten.

In einer weiteren Ausgestaltung der Erfindung kann der erste Ballon mittels eines Stegs mit dem zweiten Ballon verbunden sein, sodass der erste und der zweite Ballon durch dieselbe Ballonhülle ausgebildet werden. Unter Steg ist somit die Ballonhülle zu verstehen, die den Schaft in dem Bereich zwischen dem distalen Ende des ersten Ballons und dem proximalen Ende des zweiten Ballons umhüllt. Der Durchmesser des Schaftes inklusive Steg ist in einer solchen Ausgestaltung, bei Ballons im inflatierten Zustand um maximal 15 %, bevorzugt maximal 10 %, ganz besonders bevorzugt maximal 5 %, insbesondere 0,5 % größer als der Schaftdurchmesser exklusive Steg in diesem Bereich.

Erfindungsgemäß können die Ballons diverse Formen aufweisen. In einer vorteilhaften Ausgestaltung der Erfindung weist der zweite, distale Ballon im inflatierten Zustand eine Form auf, bei der der Durchmesser des proximalen Endes des Ballons geringer ist, als der Durchmesser des distalen Endes des Ballons. Bevorzugt weist der zweite Ballon die Form eines Kegels, eines Kegelstumpfes, einer Pyramide oder eines Pyramidenstumpfes auf, wobei die Spitze beziehungsweise die Deckfläche in Richtung des proximalen Endes des Schafts zeigt. Solche oder ähnliche Formen bieten den Vorteil, dass sich das distale Ende des Katheders bei Inflation des Ballons automatisch in die Paukenhöhle hineinzieht.

In einer vorteilhaften Ausgestaltung der Erfindung erstreckt sich der zweite Ballon im inflatierten Zustand entlang der Längsachse des Schafts teilweise über das distale Ende des Schafts hinaus. Bei einem solchen Katheter sind die Austrittsöffnung des Applikationslumens und die Eintrittsöffnung des Entlüftungslumens, bei Betrachtung des Schafts senkrecht zur Längsachse, zurückgezogen hinten dem Ballonabschnitt, der über das distale Ende des Katheters hinausgeht, angeordnet. Hierdurch ist eine weiter verbesserte Schutzwirkung gegeben, sodass die Gefahr einer Verletzung der Gehörknöchelchen minimiert ist.

Der erfindungsgemäße Schaft kann zudem Röntgenmarker aufweisen, mit denen die Position des Schafts bestimmt werden kann. Bevorzugt befinden sich diese Marker am distalen Ende des Schafts, zwischen dem ersten und dem zweiten Ballon und/oder proximal des zweiten Ballons.

Somit ist die Erfindung gerichtet auf einen Katheter zur Applikation eines Mediums in das Mittelohr umfassend einen Schaft mit einem distalen und einem proximalen Ende, einen inflatierbaren ersten Ballon, wobei das Innere des ersten Ballons mit einem ersten Inflationslumen in Verbindung steht, einen inflatierbaren zweiten Ballon, der distal zum ersten Ballon angeordnet ist, wobei das Innere des zweiten Ballons mit einem zweiten Inflationslumen in Verbindung steht, ein Applikationslumen mit einer Eintrittsöffnung, einer Austrittsöffnung und einem Mittelabschnitt, der sich von der Eintrittsöffnung bis zur Austrittsöffnung erstreckt, wobei die Eintrittsöffnung außerhalb des Schafts angeordnet ist, sich der Mittelabschnitt zumindest teilweise durch den Schaft erstreckt und die Austrittsöffnung distal des proximalen Endes des zweiten Ballons angeordnet ist, und ein Entlüftungslumen mit einer Eintrittsöffnung, einer Austrittsöffnung und einem Mittelabschnitt, der sich von der Eintrittsöffnung bis zur Austrittsöffnung erstreckt, wobei die Eintrittsöffnung distal des proximalen Endes des zweiten Ballons angeordnet ist, sich der Mittelabschnitt zumindest teilweise durch den Schaft erstreckt und die Austrittsöffnung außerhalb des Schafts angeordnet ist,
- bei dem das erste Inflationslumen und das zweite Inflationslumen nicht miteinander in Fluidverbindung stehen,
- bei dem der erste und der zweite Ballon mittels je eines Lumens inflatierbar sind,
- bei dem Austrittsöffnung des Applikationslumens und die Eintrittsöffnung des Entlüftungslumen am distalen Ende des Schaftes angeordnet sind,
- bei dem der zweite Ballon am distalen Ende des Schaftes angeordnet ist,
- bei dem das Entlüftungslumen einen größeren Durchmesser aufweist als das Applikationslumen,
- bei dem der Schaftdurchmesser weniger als 2 mm, besonders bevorzugt weniger als 1,5 mm, besonders bevorzugt weniger als 1,2 mm, insbesondere weniger als 1 mm beträgt,
- bei dem der erste und/oder zweite Ballon mit weniger als 1 bar, bevorzugt weniger als 0,75 bar, ganz besonders bevorzugt weniger als 0,5 bar Überdruck inflatierbar ist,
- bei dem der erste Ballon im inflatierten Zustand eine Länge von 5 bis 30 mm, bevorzugt 8 bis 20 mm, besonders bevorzugt 10 bis 16 mm, und/oder einen Durchmesser von 3 bis 12 mm, bevorzugt von 5-10 mm, besonders bevorzugt 7-8 mm aufweist,
- bei dem der zweite Ballon im inflatierten Zustand eine Länge von 2 bis 8 mm, bevorzugt von 4 bis 6 mm, und/oder einen Durchmesser von 2 bis 8 mm, bevorzugt 4 bis 6 mm aufweist,
- bei dem der erste Ballon 3-10 mm, bevorzugt 5-8 mm von dem zweiten Ballon beabstandet ist,
- bei dem der erste und der zweite Ballon mittels eines Stegs miteinander verbunden sind,
- bei dem der zweite Ballon im inflatierten Zustand eine Form aufweist, bei der der Durchmesser des proximalen Endes des Ballons geringer ist, als der Durchmesser des distalen Endes des Ballons, und/oder
- bei dem sich der zweite Ballon im inflatierten Zustand entlang der Längsachse des Schafts teilweise über das distale Ende des Schafts hinaus erstreckt.

Zur Lösung der Aufgabe ist somit ein Katheter zur Applikation eines Mediums vorgesehen, umfassend ein erstes Inflationslumen, ein zweites Inflationslumen, ein Applikationslumen und ein Entlüftungslumen, einen ersten Ballon, einen zweiten Ballon, der distal zum ersten Ballon angeordnet ist, wobei der erste Ballon mittels des ersten Inflationslumens und der zweite Ballon mittels des zweiten Inflationslumens inflatierbar sind, und wobei das Applikationslumen und das Entlüftungslumen bis zum distalen Ende des Katheters verlaufen.

Die Erfindung wird im Folgenden anhand der Figuren nochmals erläutert.
**Fig. 1** zeigt einen Teilausschnitt einer Ausführungsform des erfindungsgemäßen Katheters.
**Fig. 2** zeigt eine schematische Darstellung eines Querschnitts entlang der Längsachse einer Ausführungsform des erfindungsgemäßen Katheters.
**Fig. 3** zeigt eine schematische Darstellung eines Querschnitts entlang der Längsachse einer Ausführungsform des erfindungsgemäßen Katheters.

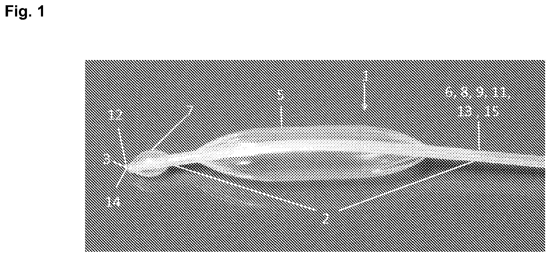

**Fig. 1** zeigt einen Ausschnitt einer Ausführungsform des erfindungsgemäßen Katheters **1,** der einen Schaft **2** mit einem distalen Ende des Schafts **3** sowie einem nicht dargestellten proximalen Ende des Schafts **4** umfasst. In der dargestellten Ausführungsform umfasst der Katheter **1** einen inflatierbaren ersten Ballon **5,** dessen Inneres mit einem ersten Inflationslumen **6** in Verbindung steht. Distal des ersten Ballons **5** ist ein inflatierbarer zweiter Ballon **7** angeordnet, dessen Inneres mit einem zweiten Inflationslumen **8** in Verbindung steht. Das erste und das zweite Inflationslumen verlaufen in dem dargestellten Katheterabschnitt innerhalb des Schafts **2.** Zudem weist der Katheter ein Applikationslumen **9** mit einer nicht dargestellten Eintrittsöffnung **10** außerhalb des Schafts, einer Austrittsöffnung **12** und einem Mittelabschnitt **11,** der sich von der Eintrittsöffnung bis zur Austrittsöffnung erstreckt, auf. Die Austrittsöffnung **12** des Applikationslumens ist distal des proximalen Endes des zweiten Ballons **7** angeordnet. Ebenso umfasst der Katheter **1** ein Entlüftungslumen **13** mit einer Eintrittsöffnung **14,** die distal des proximalen Endes des zweiten Ballons **7** angeordnet ist. Zwischen der Eintrittsöffnung **14** und der nicht dargestellten Austrittsöffnung **16** außerhalb des Schafts erstreckt sich der Mittelabschnitt **15.**

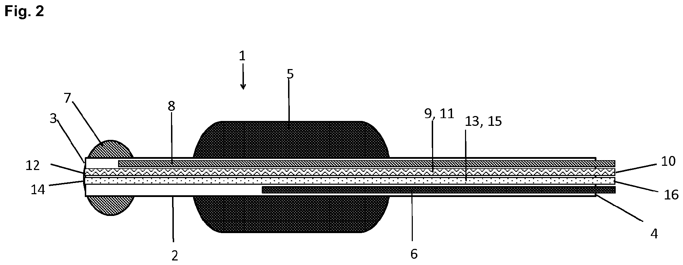

**Fig. 2** zeigt eine schematische Querschnittsdarstellung eines erfindungsgemäßen Katheters **1** entlang der Längsachse des Katheters **1,** der einen Schaft **2** mit einem distalen Ende **3** und einem proximalen Ende **4** umfasst. Der Katheter **1** umfasst ferner einen ersten Ballon **5,** der mittels eines ersten Inflationslumens **6** inflatierbar ist, sowie einen zweiten Ballon **7,** der mittels eines zweiten Inflationslumens **8** inflatierbar ist. Distal des proximalen Endes des zweiten Ballons **7** sind die Austrittsöffnung des Applikationslumens **9** und die Eintrittsöffnung des Entlüftungslumens **14** angeordnet. Von der Austrittsöffnung des Applikationslumens **12** erstreckt sich der Mittelabschnitt des Applikationslumens **11** durch das proximale Ende des Schafts und endet in der Eintrittsöffnung **10.** Von der Eintrittsöffnung des Entlüftungslumens **14** erstreckt sich der Mittelabschnitt des Entlüftungslumens **15** durch das proximale Ende des Schafts und endet in der Austrittsöffnung **16.**

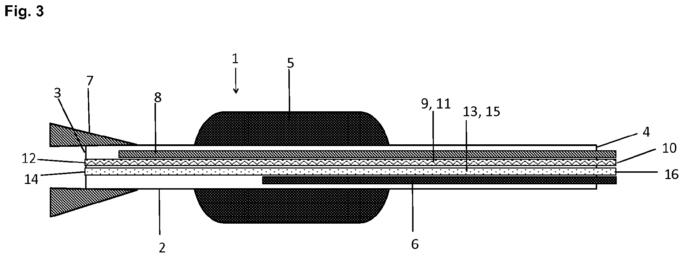

**Fig. 3** zeigt eine schematische Querschnittsdarstellung eines erfindungsgemäßen Katheters **1** wie in **Fig. 2** dargestellt, bei dem bei dem der zweite Ballon **7** im inflatierten Zustand eine Form aufweist, bei der der Durchmesser des proximalen Endes des Ballons geringer ist, als der Durchmesser des distalen Endes des Ballons. Der zweite Ballon **7** erstreckt sich im inflatierten Zustand, wie dargestellt, in Längsrichtung des Katheters teilweise über das distale Ende des Schafts **3** hinaus.

### Bezugszeichenliste

- 1: Katheter
- 2: Schaft
- 3: Distales Ende des Schafts
- 4: Proximales Ende des Schafts
- 5: Erster Ballon
- 6: Erstes Inflationslumen
- 7: Zweiter Ballon
- 8: Zweites Inflationslumen
- 9: Applikationslumen
- 10: Eintrittsöffnung des Applikationslumens
- 11: Mittelabschnitt des Applikationslumens
- 12: Austrittsöffnung des Applikationslumens
- 13: Entlüftungslumen
- 14: Eintrittsöffnung des Entlüftungslumens
- 15: Mittelabschnitt des Entlüftungslumens
- 16: Austrittsöffnung des Entlüftungslumens

## Patentansprüche

1. Katheter (1) zur Applikation eines Mediums in das Mittelohr, umfassend
einen Schaft (2) mit einem distalen (3) und einem proximalen (4) Ende,
einen inflatierbaren ersten Ballon (5), wobei das Innere des ersten Ballons mit einem ersten Inflationslumen (6) in Verbindung steht,
einen inflatierbaren zweiten Ballon (7), der distal zum ersten Ballon angeordnet ist,
wobei das Innere des zweiten Ballons mit einem zweiten Inflationslumen (8) in Verbindung steht,
ein Applikationslumen (9) mit einer Eintrittsöffnung (10), einer Austrittsöffnung (12) und einem Mittelabschnitt (11), der sich von der Eintrittsöffnung bis zur Austrittsöffnung erstreckt,
wobei die Eintrittsöffnung außerhalb des Schafts angeordnet ist, sich der Mittelabschnitt zumindest teilweise durch den Schaft erstreckt und die Austrittsöffnung distal des proximalen Endes des zweiten Ballons angeordnet ist, und
ein Entlüftungslumen (13) mit einer Eintrittsöffnung (14), einer Austrittsöffnung (16) und einem Mittelabschnitt (15), der sich von der Eintrittsöffnung bis zur Austrittsöffnung erstreckt,
wobei die Eintrittsöffnung distal des proximalen Endes des zweiten Ballons angeordnet ist, sich der Mittelabschnitt zumindest teilweise durch den Schaft erstreckt und die Austrittsöffnung außerhalb des Schafts angeordnet ist,
wobei die Austrittsöffnung des Applikationslumens und die Eintrittsöffnung des Entlüftungslumen am distalen Ende des Schaftes angeordnet sind.

2. Katheter nach Anspruch 1, bei dem das erste Inflationslumen und das zweite Inflationslumen nicht miteinander in Fluidverbindung stehen.

3. Katheter nach einem der Ansprüche 1 bis 2, bei dem der zweite Ballon am distalen Ende des Schaftes angeordnet ist.

4. Katheter nach einem der Ansprüche 1 bis 3, bei dem das Entlüftungslumen einen größeren Durchmesser aufweist als das Applikationslumen.

5. Katheter nach einem der Ansprüche 1 bis 4, bei dem der Schaftdurchmesser weniger als 2 mm, bevorzugt weniger als 1,5 mm, besonders bevorzugt weniger als 1,2 mm, insbesondere weniger als 1 mm beträgt.

6. Katheter nach einem der Ansprüche 1 bis 5, bei dem der erste und/oder zweite Ballon mit weniger als 1 bar, bevorzugt weniger als 0,75 bar, ganz besonders bevorzugt weniger als 0,5 bar Überdruck inflatierbar ist.

7. Katheter nach einem der Ansprüche 1 bis 6, bei dem der erste Ballon im inflatierten Zustand eine Länge von 5 bis 30 mm, bevorzugt 8 bis 20 mm, besonders bevorzugt 10 bis 16 mm, und/oder der erste Ballon im inflatierten Zustand einen Durchmesser von 3 bis 12 mm, bevorzugt von 5-10 mm, besonders bevorzugt 7-8 mm aufweist.

8. Katheter nach einem der Ansprüche 1 bis 7, bei dem der zweite Ballon im inflatierten Zustand eine Länge von 2 bis 8 mm, bevorzugt von 4 bis 6 mm, und/oder der zweite Ballon im inflatierten Zustand einen Durchmesser von 2 bis 8 mm, bevorzugt 4 bis 6 mm aufweist.

9. Katheter nach einem der Ansprüche 1 bis 8, bei dem der erste Ballon 3-10 mm, bevorzugt 5-8 mm, von dem zweiten Ballon beabstandet ist.

10. Katheter nach einem der Ansprüche 1 bis 9, bei dem der erste und der zweite Ballon mittels eines Stegs miteinander verbunden sind.

11. Katheter nach einem der Ansprüche 1 bis 10, bei dem der zweite Ballon im inflatierten Zustand eine Form aufweist, bei der der Durchmesser des proximalen Endes des Ballons geringer ist, als der Durchmesser des distalen Endes des Ballons.

12. Katheter nach einem der Ansprüche 1 bis 11, bei dem sich der zweite Ballon im inflatierten Zustand entlang der Längsachse des Schafts teilweise über das distale Ende des Schaftes hinaus erstreckt.

## Claims

1. A catheter (1) for applying a medium into the middle ear, comprising a shaft (2) having a distal (3) and a proximal (4) end,
an inflatable first balloon (5), the interior of the first balloon being in communication with a first inflation lumen (6),
an inflatable second balloon (7) which is arranged distally in relation to the first balloon, the interior of the second balloon being in communication with a second inflation lumen (8),
an application lumen (9) having an inlet opening (10), an outlet opening (12) and a center section (11) that extends from the inlet opening to the outlet opening, the inlet opening being arranged outside of the shaft, the center section extending at least partially through the shaft and the outlet opening being arranged distally in relation to the proximal end of the second balloon, and
a vent lumen (13) having an inlet opening (14), an outlet opening (16) and a center section (15) that extends from the inlet opening to the outlet opening, the inlet opening being arranged distally in relation to the proximal end of the second balloon, the center section extending at least partially through the shaft and the outlet opening being arranged outside of the shaft, wherein the outlet opening of the application lumen and the inlet opening of the vent lumen are arranged at the distal end of the shaft.

2. The catheter of claim 1, wherein the first inflation lumen and the second inflation lumen are not in fluid communication with each other.

3. The catheter of any one of claims 1 to 2, wherein the second balloon is arranged at the distal end of the shaft.

4. A catheter according to any one of claims 1 to 3, wherein the vent lumen has a larger diameter than the application lumen.

5. The catheter according to any one of claims 1 to 4, wherein the shaft diameter is less than 2 mm, preferably less than 1.5 mm, particularly preferably less than 1.2 mm, in particular less than 1 mm.

6. The catheter according to any one of claims 1 to 5, wherein the first and/or second balloons are inflatable with less than 1 bar, preferably less than 0.75 bar, very particularly preferably less than 0.5 bar gauge.

7. The catheter according to any one of claims 1 to 6, wherein the first balloon in the inflated state has a length of 5 to 30 mm, preferably 8 to 20 mm, particularly preferably 10 to 16 mm, and/or the first balloon in the inflated state has a diameter of 3 to 12 mm, preferably 5-10 mm, particularly preferably 7-8 mm.

8. The catheter according to any one of claims 1 to 7, wherein the second balloon in the inflated state has a length of 2 to 8 mm, preferably 4 to 6 mm, and/or the second balloon in the inflated state has a diameter of 2 to 8 mm, preferably 4 to 6 mm.

9. The catheter according to any one of claims 1 to 8, wherein the first balloon is spaced 3-10 mm, preferably 5-8 mm from the second balloon.

10. The catheter according to any one of claims 1 to 9, wherein the first and second balloons are interconnected by means of a web.

11. The catheter according to any one of claims 1 to 10, wherein the second balloon in the inflated state has a shape in which the diameter of the proximal end of the balloon is less than the diameter of the distal end of the balloon.

12. The catheter of any one of claims 1 to 11, wherein the second balloon in the inflated state extends partially beyond the distal end of the shaft along the longitudinal axis of the shaft.

## Revendications

1. Cathéter (1), destiné à appliquer un fluide dans l'oreille moyenne, comprenant
une tige (2) pourvue d'une extrémité distale (3) et d'une extrémité proximale (4),
un premier ballonnet gonflable (5),
l'intérieur du premier ballonnet étant en liaison avec une première lumière de gonflage (6),
un deuxième ballonnet gonflable (7), qui est placé de manière distale par rapport au premier ballonnet,
l'intérieur du deuxième ballonnet étant en liaison avec une deuxième lumière gonflable (8),
une lumière d'application (9) pourvue d'un orifice d'entrée (10), d'un orifice de sortie (12) et d'un segment médian (11), qui s'étend de l'orifice d'entrée jusqu'à l'orifice de sortie,
l'orifice d'entrée étant placé à l'extérieur de la tige, le segment médian s'étendant au moins en partie à travers la tige et l'orifice de sortie étant placé de manière distale par rapport à l'extrémité proximale du deuxième ballonnet, et
une lumière de purge (13) pourvue d'un orifice d'entrée (14), d'un orifice de sortie (16) et d'un segment médian (15), qui s'étend de l'orifice d'entrée jusqu'à l'orifice de sortie,
l'orifice d'entrée étant placé de manière distale par rapport à l'extrémité proximale du deuxième ballonnet, le segment médian s'étendant au moins en partie à travers la tige et l'orifice de sortie étant placé à l'extérieur de la tige,
l'orifice de sortie de la lumière d'application et l'orifice d'entrée de la lumière de purge étant placés sur l'extrémité distale de la tige.

2. Cathéter selon la revendication 1, sur lequel la première lumière de gonflage et la deuxième lumière de gonflage ne se trouvent pas en liaison fluidique mutuelle.

3. Cathéter selon l'une quelconque des revendications 1 à 2, sur lequel le deuxième ballonnet est placé sur l'extrémité distale de la tige.

4. Cathéter selon l'une quelconque des revendications 1 à 3, sur lequel la lumière de purge présente un diamètre supérieur à celui de la lumière d'application.

5. Cathéter selon l'une quelconque des revendications 1 à 4, sur lequel le diamètre de la tige est de moins de 2 mm, de préférence de moins de 1,5 mm, de manière particulièrement préférentielle de moins de 1,2 mm, notamment de moins de 1 mm.

6. Cathéter selon l'une quelconque des revendications 1 à 5, sur lequel le premier et/ou le deuxième ballonnet est gonflable à moins de 1 bar, de préférence à moins de 0,75 bar, de manière particulièrement préférentielle, à moins de 0,5 bar de surpression.

7. Cathéter selon l'une quelconque des revendications 1 à 6, sur lequel à l'état gonflé, le premier ballonnet présente une longueur de 5 à 30 mm, de préférence de 8 à 20 mm, de manière particulièrement préférentielle, de 10 à 16 mm, et/ou à l'état gonflé, le premier ballonnet présente un diamètre de 3 à 12 mm, de préférence de 5 à 10 mm, de manière particulièrement préférentielle de 7 à 8 mm.

8. Cathéter selon l'une quelconque des revendications 1 à 7, sur lequel, à l'état gonflé, le deuxième ballonnet présente une longueur de 2 à 8 mm, de préférence de 4 à 6 mm, et/ou à l'état gonflé, le deuxième ballonnet présente un diamètre de 2 à 8 mm, de préférence de 4 à 6 mm.

9. Cathéter selon l'une quelconque des revendications 1 à 8, sur lequel le premier ballonnet est écarté de 3 à 10 mm, de préférence de 5 à 8 mm du deuxième ballonnet.

10. Cathéter selon l'une quelconque des revendications 1 à 9, sur lequel le premier et le deuxième ballonnets sont reliés l'un à l'autre au moyen d'un listel.

11. Cathéter selon l'une quelconque des revendications 1 à 10, sur lequel à l'état gonflé, le deuxième ballonnet présente une forme sur laquelle le diamètre de l'extrémité proximale du ballonnet est inférieur au diamètre de l'extrémité distale du ballonnet.

12. Cathéter selon l'une quelconque des revendications 1 à 11, sur lequel à l'état gonflé, le deuxième ballonnet s'étend le long de l'axe longitudinal de la tige, partiellement au-delà de l'extrémité distale de la tige.
